# EUROPEAN PATENT SPECIFICATION

(11) **EP 3 178 521 B1**
(45) Date of publication and mention of the grant of the patent: **02.06.2021**
(21) Application number: 16200019.4
(22) Date of filing: 22.11.2016
(51) Int. Cl.: A61N 1/05, A61N 1/362, A61N 1/378

(54) **DEVICE FOR THE TREATMENT OF THE INTEGRATED HEART RATE**
VORRICHTUNG ZUR BEHANDLUNG DER INTEGRIERTEN HERZFREQUENZ
DISPOSITIF POUR LE TRAITEMENT DU TAUX DE COEUR INTÉGRÉ

(30) Priority: 24.11.2015 IT UB20155860
(43) Date of publication of application: 14.06.2017
(73) Proprietor: Fiab S.P.A., 50039 Vicchio (IT)
(72) Inventor: CALABRO', Alberto, 50139 Firenze (IT); FASANO, Antonio, 50139 FIRENZE (IT)
(74) Representative: Paparo, Aldo

(56) References cited:
- GB-A- 2 370 509
- US-A- 4 134 408
- US-A1- 2006 085 051
- US-A1- 2010 114 215
- US-A1- 2010 114 235
- US-A1- 2011 152 747

## Description

### DESCRIPTION

This invention concerns a device for the temporary treatment of the heart rate, also known as an external pacemaker, integrated with a supply system that makes it possible to increase the operation of the device and to allow more time to replace the main battery of the system compared with prior art devices.

Currently, temporary pacemakers present a supply circuit that is in turn powered by a battery which, also in view of the dimensions of the device, has a naturally limited life, which can for example be twelve hours. The limited life of the battery means that it needs to be regularly replaced, with the risks involved each time it is replaced because of the need to keep the pacemaker operational while it is being replaced.

Known external pacemakers are described in the following prior documents: GB 2370509, US 2006/085051 and US 2011/152747.

The aim of this invention is to provide a device for the temporary treatment of the heart rate, which makes it possible to increase, with respect to prior art devices, the time interval available for replacement of the battery of the device.

Currently, while the battery is being replaced, the supply circuit of the device is connected to an accessory system that makes it possible to keep the pacemaker operational for a fairly short period of time during which it is necessary to carry out the replacement of the battery.

This accessory system, which can for example comprise capacitors, obliges operatives to carry out replacement of the battery in a very short period of time, with the risk (for example for a slight hitch) of not being able to replace the battery in time, and thus of producing serious consequences for the patient.

Another aim of this invention is to provide a device for the temporary treatment of the heart rate that makes it possible to substantially increase the functioning time of the device, beyond the time limits of a complex procedure that are needed and which at the same time provides a guarantee against an, albeit highly improbable, sudden malfunction of the battery.

The aims of this invention are achieved by means of a device as defined in claim 1.

The control unit is preferably configured to ensure the auxiliary battery alternately supplies the main battery or the circuit or both, on the basis of the charge level of the batteries.

The control unit is preferably configured to ensure the photovoltaic panel alternately supplies the main battery or the auxiliary battery or both, on the basis of the charge level of each of the batteries.

The supply system preferably comprises a first switching system designed to be connected to at least one of the batteries and to the circuit.

Both the main battery and auxiliary battery are preferably designed to supply the circuit by the interposition of the first switching system.

The control unit is preferably configured to control the first switching system, so that it is possible to select whether the main battery or the auxiliary battery supplies the circuit.

The auxiliary battery is preferably designed to supply the main battery and/or the circuit by the interposition of the first switching system.

In this way, by controlling the first switching system, the control unit can select whether the auxiliary battery or the main battery supplies the circuit. The supply system preferably comprises a second switching system designed to be connected to the panel and to at least one of the batteries. The panel is preferably designed to supply the auxiliary battery and/or the main battery by the interposition of the second switching system.

The control unit is preferably configured to control the second switching system in order to select whether the panel supplies the main battery, the auxiliary battery or both.

The features of this invention are described in detail below relating to a possible embodiment of the invention to be considered by way of a nonlimiting example of the more general concepts claimed.

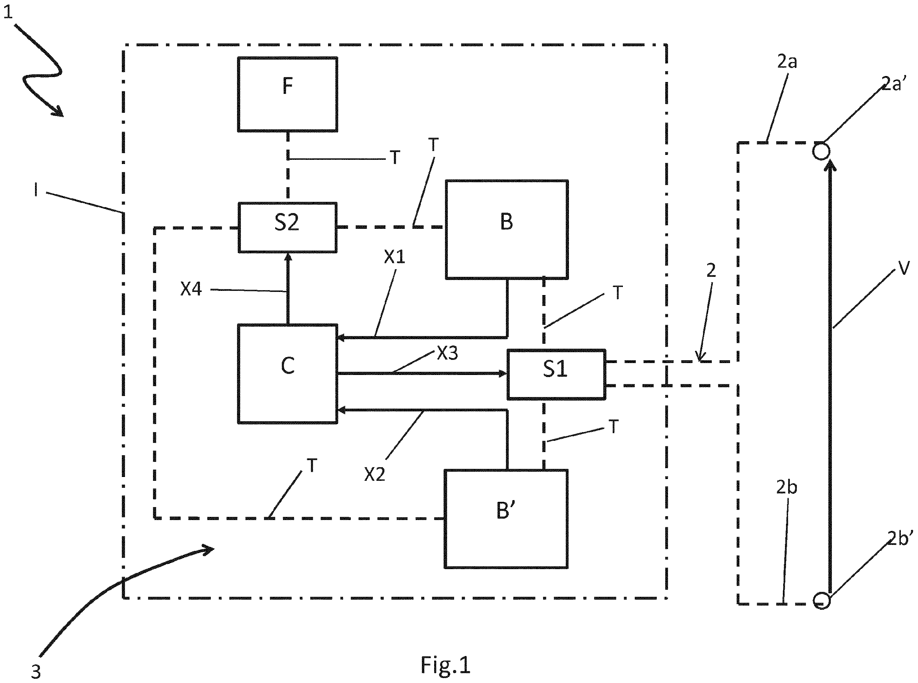

The following detailed description refers to the attached drawing, in which figure 1 is a block diagram representing the functioning of a possible embodiment of the invention.

In Figure 1 the numeral 1 is a block diagram of a device according to a possible embodiment of this invention. The device 1 comprises means for stimulating the heart rate, which are not shown. The device 1 comprises an electric circuit 2 connected to the means for stimulating the heart rate. The circuit 2 is designed to activate the means for stimulating the heart rate, and thus to keep them in working condition.

The device 1 comprises a supply system 3 for the circuit 2.

The box marked I represents a casing that comprises and/or contains at least part of the supply system 3.

The circuit 2 can comprise, for example, cables 2a and 2b which act as electrodes. These cables are, for example, connected to the components inside the casing I, by connectors.

The voltage charge required by the pacemaker is marked by an arrow V, which indicates a voltage between the clamps 2a' and 2b' of the cables 2a and 2b.

The supply system 3 comprises a main battery B which can be connected to the circuit 2 to supply it. The supply system 3 advantageously comprises at least one line of transport T of electrical energy which allows the circuit 2 to be supplied by the main battery B.

In general, in the attached drawing, the transport lines of electrical energy are all indicated with the letter T. Each of these transport lines T is represented by a dashed line between the boxes relative to the components between which the current and/or electrical energy passes. The supply system 3 comprises a photovoltaic panel F which can be connected to the main battery B to supply it.

In this description, where there is a reference to the supply of a battery, this supply can be intended as also meaning the recharge of the battery, both when the device 1 is operational and when it is not operational.

The photovoltaic panel F is designed, when it is connected to the main battery B, to supply the main battery B even when the device 1 is functioning.

The supply system 3 thus advantageously comprises at least one transport line T of electrical energy between the photovoltaic panel F and the main battery B.

The supply system 3 comprises a control unit.

The supply system 3 comprises an auxiliary battery B' connectable to the circuit 2 to supply the circuit 2 and/or to the panel F to be supplied by the panel.

The supply system 3 advantageously comprises at least one transport line of electrical energy which allows the circuit 2 to be supplied by the auxiliary battery B'.

The supply system 3 advantageously comprises at least one transport line of electrical energy which allows the auxiliary battery B' to be supplied by the panel F.

The control unit is configured to ensure the circuit 2 is supplied alternately by the main battery B or by the auxiliary battery B'.

The control unit is configured to select whether the circuit 2 is supplied by the main battery B and the auxiliary battery B', on the basis of the charge level of the main battery B and the auxiliary battery B'.

The supply system 3 thus advantageously comprises at least one transfer line X1 between the main battery B and the control unit C and/or at least one transfer line X2 between the auxiliary battery B' and the control unit C. The transfer line X1 between the main battery B and the control unit C schematically represents electronic means designed to transport a signal indicating the charge level of the main battery B. The transfer line X2 between the auxiliary battery B' and the control unit C schematically represents electronic means designed to transport a signal indicating the charge level of the auxiliary battery B'.

To ensure that the control unit C can effectively select whether the circuit is supplied by the main battery B or the auxiliary battery B', the supply system 3 comprises a first switching system S1 designed to be connected to at least either the main battery B or the auxiliary battery B' and to the circuit 2.

Both the main battery B and the auxiliary battery B' are designed to supply the circuit 2 by the interposition of the first switching system S1.

The system 3 thus comprises lines for the transport T of electrical energy between the main battery B and the first switching system S1, between the auxiliary battery B' and the first switching system S1, and between the first switching system S1 and the circuit 2.

The control unit C is configured to control the first switching system S1 in order to select whether the circuit 2 is supplied by the main battery B or the auxiliary battery B'. The system 3 thus comprises the transfer line X3 which schematically represents electronic means designed to transport a signal designed to control the switching system S1 by the control unit C.

In the embodiment shown, the control unit can be configured to ensure the auxiliary battery B' alternately supplies the main battery B or the circuit 2 or both, on the basis of the charge level of the main battery B and the auxiliary battery B'.

In the embodiment shown, the auxiliary battery B' is designed to supply the main battery B and/or the circuit 2 by interposition of the first switching system S1.

In this way, by controlling the first switching system S1, the control unit C can also select whether the circuit 2 is supplied by the auxiliary battery B' or the main battery B or both.

In the embodiment shown, the control unit C is configured to ensure the photovoltaic panel F alternately supplies the main battery B or the auxiliary battery B' or both, on the basis of the charge level of the main battery B and the auxiliary battery B'.

To ensure that the control unit C can effectively select whether the photovoltaic panel F alternately supplies the main battery B or the auxiliary battery B' or both, the supply system 3 comprises a second switching system S2 designed to be connected to at least either the auxiliary battery B' or the main battery B and to the photovoltaic panel F.

The panel F is designed to supply the auxiliary battery B' and/or the main battery B by interposition of the second switching system S2.

The system 3 thus comprises lines for the transport T of electrical energy between the panel F and the second switching system S2, between the second switching system S2 and the main battery B, and between the second switching system S2 and the auxiliary battery B'.

The control unit C is configured to control the second switching system S2 in order to select whether the panel F supplies the auxiliary battery B' and/or the main battery B. The system 3 thus comprises the transfer line X4 which schematically represents electronic means designed to transport a signal designed to control the second switching system S2 by the control unit C.

The control unit C could be programmed to that the auxiliary battery B' supplies the circuit 2 when the charge level of the main battery B falls below a certain preset value.

The control unit C could be programmed to that the panel F supplies the main battery B while this battery is supplying the circuit 2, and/or also when the auxiliary battery B' is supplying the circuit 2.

The control unit C could be programmed so that when the charge level of the main battery B rises or is above a certain level, the panel F starts to supply the auxiliary battery B'.

The panel F is located in a position designed to receive the light from environment external to the device 1, and thus on an outer surface of the casing I.

The invention achieves the proposed aims, and makes it possible to increase the life of a device for the temporary and/or external treatment of the heart rate before it is necessary to change at least one battery of the device.

The invention also makes it possible to increase the safety while replacing the main battery, by means of the presence of a back-up or auxiliary battery which makes it possible to extend the interval of time during which replacement of the main battery can be carried out.

## Claims

1. An external device (1) for temporarily treating the heart rate, comprising means for stimulating the heart rate, a circuit (2) connected to the stimulation means to actuate them, and a system (3) for supplying the circuit, comprising:
a main battery (B) connectable to the circuit to supply it;
a photovoltaic panel (F) connectable to the main battery to supply the main battery during the operation of the device itself;
an auxiliary battery (B') connectable to the circuit (2) to supply it and to
the photovoltaic panel (F) to be supplied by the photovoltaic panel; and
a control unit (C);
wherein the control unit is configured to ensure the circuit is supplied alternately by the main battery or by the auxiliary battery, on the basis of the charge level of each of the batteries.

2. The device according to claim 1, wherein the system comprises a first switching system (S1) designed to be connected to at least one of the batteries and to the circuit, the main battery and auxiliary battery each being designed to supply the circuit by the interposition of the first switching system, and wherein the control unit is configured to control the first switching system.

3. The device according to claim 1 or 2, wherein the control unit (C) is configured to ensure the auxiliary battery alternately supplies the main battery or the circuit or both, on the basis of the charge level of the batteries.

4. The device according to claim 3, wherein the auxiliary battery is designed to supply the main battery and/or the circuit by the interposition of the first switching system.

5. The device according to any of previous claims, wherein the control unit is configured to ensure the photovoltaic panel alternately supplies the main battery or the auxiliary battery or both, on the basis of the charge level of each of the batteries.

6. The device according to claim 5, wherein the system comprises a second switching system (S2) designed to be connected to the photovoltaic panel and to at least one of the batteries, the photovoltaic panel being designed to supply the auxiliary battery and/or the main battery by the interposition of the second switching system, and wherein the control unit is configured to control the second switching system.

## Patentansprüche

1. Externe Vorrichtung (1) zur vorübergehenden Behandlung der Herzfrequenz, umfassend Mittel zum Stimulieren der Herzfrequenz, einen Stromkreis (2), der mit den Stimulationsmitteln verbunden ist, um sie zu betätigen, und ein System (3) zum Versorgen des Stromkreises, umfassend:
eine Hauptbatterie (B), die mit dem Stromkreis verbunden werden kann, um ihn zu versorgen;
ein Photovoltaik-Panel (F), das mit der Hauptbatterie verbunden werden kann, um die Hauptbatterie während des Betriebs der Vorrichtung selbst zu versorgen;
eine Hilfsbatterie (B'), die mit dem Stromkreis (2) zu deren Versorgung und mit dem Photovoltaik-Modul verbunden werden kann; und eine Steuereinheit (C);
wobei die Steuereinheit ausgebildet ist, um sicherzustellen, dass der Stromkreis abwechselnd von der Hauptbatterie oder von der Hilfsbatterie auf der Basis des Ladezustands einer jeden der Batterien versorgt wird.

2. Vorrichtung nach Anspruch 1, wobei das System ein erstes Schaltsystem (S1) umfasst, das so ausgelegt ist, dass es mit mindestens einer der Batterien und mit dem Stromkreis verbunden ist, wobei die Hauptbatterie und die Hilfsbatterie jeweils so ausgelegt sind, dass sie den Stromkreis durch Zwischenschaltung des ersten Schaltsystems versorgen, und wobei die Steuereinheit ausgebildet ist, um das erste Schaltsystem zu steuern.

3. Vorrichtung nach Anspruch 1 oder 2, wobei die Steuereinheit (C) ausgebildet ist, um sicherzustellen, dass die Hilfsbatterie abwechselnd die Hauptbatterie oder den Stromkreis oder beides auf der Basis des Ladezustands der Batterien versorgt.

4. Vorrichtung nach Anspruch 3, wobei die Hilfsbatterie so ausgelegt ist, dass sie die Hauptbatterie und/oder den Stromkreis durch Zwischenschaltung des ersten Schaltsystems versorgt.

5. Vorrichtung nach einem der vorhergehenden Ansprüche, wobei die Steuereinheit ausgebildet ist, um sicherzustellen, dass das Photovoltaik-Panel abwechselnd die Hauptbatterie oder die Hilfsbatterie oder beides auf der Basis des Ladezustands einer jeden der Batterien versorgt.

6. Vorrichtung nach Anspruch 5, wobei das System ein zweites Schaltsystem (S2) umfasst, das zur Verbindung mit dem Photovoltaik-Panel und mit mindestens einer der Batterien ausgelegt ist, wobei das Photovoltaik-Panel zur Versorgung der Hilfsbatterie und/oder der Hauptbatterie durch Zwischenschaltung des zweiten Schaltsystems ausgelegt ist, wobei die Steuereinheit ausgebildet ist, um das zweite Schaltsystem zu steuern.

## Revendications

1. Dispositif externe (1) de traitement temporaire de la fréquence cardiaque, comprenant des moyens de stimulation de la fréquence cardiaque, un circuit (2) relié aux moyens de stimulation pour les actionner, et un système (3) d'alimentation du circuit, comprenant :
une batterie principale (B) pouvant être reliée au circuit pour l'alimenter ;
un panneau photovoltaïque (F) pouvant être raccordé à la batterie principale pour alimenter la batterie principale pendant le fonctionnement du dispositif lui-même ;
une batterie auxiliaire (B') pouvant être raccordée au circuit (2) pour l'alimenter et au panneau photovoltaïque ; et une unité de commande (C) ;
dans lequel l'unité de commande est configurée pour que le circuit soit alimenté alternativement par la batterie principale ou par la batterie auxiliaire, sur la base du niveau de charge de chacune des batteries.

2. Dispositif selon la revendication 1, dans lequel le système comprend un premier système de commutation (S1) conçu pour être raccordé à au moins une des batteries et au circuit, la batterie principale et la batterie auxiliaire étant chacune conçue pour alimenter le circuit par l'interposition du premier système de commutation, et dans lequel l'unité de commande est configurée pour commander le premier système de commutation.

3. Dispositif selon la revendication 1 ou 2, dans lequel l'unité de commande (C) est configurée pour assurer que la batterie auxiliaire alimente alternativement la batterie principale ou le circuit ou les deux, sur la base du niveau de charge des batteries.

4. Dispositif selon la revendication 3, dans lequel la batterie auxiliaire est conçue pour alimenter la batterie principale et/ou le circuit par l'interposition du premier système de commutation.

5. Dispositif selon l'une quelconque des revendications précédentes, dans lequel l'unité de commande est configurée pour que le panneau photovoltaïque alimente alternativement la batterie principale ou la batterie auxiliaire ou les deux, sur la base du niveau de charge de chacune des batteries.

6. Dispositif selon la revendication 5, dans lequel le système comprend un deuxième système de commutation (S2) conçu pour être raccordé au panneau photovoltaïque et à au moins une des batteries, le panneau photovoltaïque étant conçu pour alimenter la batterie auxiliaire et/ou la batterie principale par l'interposition du deuxième système de commutation, et dans lequel l'unité de commande est configurée pour commander le deuxième système de commutation.
